(19) Europäisches Patentamt
European Patent Office
Office européen des brevets

(11) Numéro de publication: **0 373 057 A1**

(12) **DEMANDE DE BREVET EUROPEEN**

(21) Numéro de dépôt: **89403346.3**

(22) Date de dépôt: **01.12.89**

(51) Int. Cl.[5]: **C07C 51/347, C07C 57/03, C07C 67/347, C07C 69/533**

(30) Priorité: **02.12.88 FR 8815850**

(43) Date de publication de la demande: **13.06.90 Bulletin 90/24**

(84) Etats contractants désignés: **AT BE CH DE ES FR GB GR IT LI LU NL SE**

(71) Demandeur: **SANOFI**
**40, Avenue George V**
**F-75008 Paris(FR)**

(72) Inventeur: **Descamps, Marcel**
**6, rue de l'Anguille**
**Lherm F-31600 Muret(FR)**
Inventeur: **Sayac, Georges**
**1220 Chemin du Tucaut**
**Eaunes F-31600 Muret(FR)**

(74) Mandataire: **Varady, Peter et al**
**Cabinet Lavoix 2, Place d'Estienne d'Orves**
**F-75441 Paris Cedex 09(FR)**

(54) **Procédé de préparation de l'acide (propyl-2) pentène-2 oique et de ses esters.**

(57) Le procédé de préparation des isomères E des composés de formule A

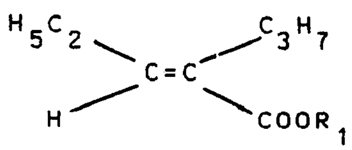

A

dans laquelle $R_1$ représente H ou un groupe alkyle en $C_1$ à $C_4$, est caractérisé selon l'invention en ce qu'on fait réagir le propionaldéhyde sur un dérivé phosphoré de formule

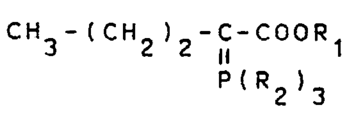

I

dans laquelle $R_1$ désigne un groupe alkyle en $C_1$ à $C_4$ et $R_2$ désigne un groupe alkyle en $C_1$ à $C_4$ ou un groupe phényle, et le cas échéant on hydrolyse le composé de formule A dans laquelle $R_1$ représente un groupe alkyle pour obtenir le composé de formule A dans laquelle $R_1$ représente H.
Industrie pharmaceutique.

EP 0 373 057 A1

## Procédé de préparation de l'acide (propyl-2) pentène-2 oïque et de ses esters

La présente invention concerne un procédé de préparation de l'isomère E de l'acide propyl-2 pentène-2 oïque et de ses esters.

Cet acide qui a été décrit notamment dans Arch. Pharm. 310(5) p. 394-403 (1977) et Journal of the American Chemical Society,93, (17) p. 4242-4247 (1971) est un métabolite de l'acide valproïque, médicament utilisé dans le traitement de l'épilepsie; ce métabolite présenterait une activité anti-épileptique non négligeable et serait moins tératogène que l'acide valproïque, et la mise au point d'un procédé de préparation donnant, avec de bons rendements, un produit pur, dépourvu d'isomères, était souhaitable pour permettre des études cliniques élargies.

En effet, les procédés décrits jusqu'à présent, que ce soit la déshydrohalogénation de l'ester valproïque alpha bromé de formule

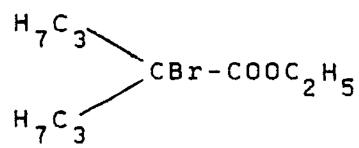

décrite dans l'article d'Arch. Pharm. précédemment cité, ou la déshydratation de l'acide alpha-hydroxylé décrite dans l'article du Journal of the American Chemical Society précédemment cité, donnent un mélange d'isomères géométriques et même de position de l'acide propyl-2 penténoïque, avec un rendement global en isomère E de l'acide propyl-2 pentène-2 oïque de 25% seulement.

On a maintenant trouvé que cet acide pouvait être préparé, avec de bons rendements, à partir de matières premières courantes, sans qu'il se forme d'isomère de position et avec très peu de son isomère géo métrique, à partir d'un ester de l'acide bromo-2 valérique. En outre, le produit brut obtenu par saponification de l'ester intermédiaire est suffisamment pur pour cristalliser spontanément.

L'invention a donc pour objet un procédé de préparation des isomères E des composés de formule A

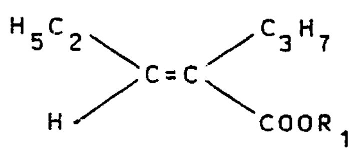

A

dans laquelle $R_1$ représente H ou un groupe alkyle en $C_1$ à $C_4$, caractérisé en ce qu'on fait réagir le propionaldéhyde sur un dérivé phosphoré de formule

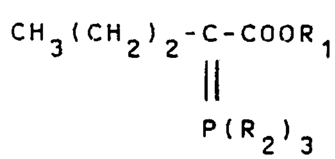

I

dans laquelle $R_1$ désigne un groupe alkyle en $C_1$ à $C_4$ et $R_2$ désigne un groupe alkyle en $C_1$ à $C_4$ ou un groupe phényle, pour obtenir un ester de l'acide propyl-2 pentène-2 oïque, en configuration E, de formule A

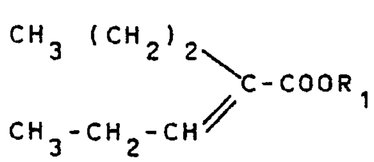

dans laquelle $R_1$ désigne un groupe alkyle en $C_1$ à $C_4$; l'acide $R_1$ = H, métabolite de l'acide valproïque, peut être ensuite isolé après hydrolyse de l'ester précédent.

Les ylides de formule I peuvent être préparés de façon classique par action d'une base sur le sel de phosphonium de formule IV

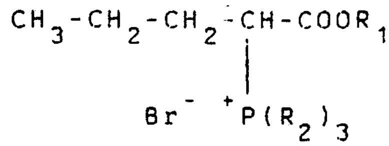

IV

résultant de la réaction d'une trialkylphosphine ou de la triphénylphosphine sur un ester de l'acide bromo-2 valérique de formule V

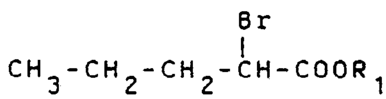

V

L'ylide de formule I dans laquelle $R_2$ représente $C_6H_5$ et $R_1$ représente $C_2H_5$ a été décrit dans Chem. Ber. 99 (4) 1198 (1966).

On fait, par exemple, réagir la triphénylphosphine à une température comprise entre 20 °C et 100 °C sur l'ester alpha-bromé en solution dans un solvant aprotique polaire qui ne comporte pas de groupes fonctionnels sur lesquels peuvent réagir des phosphines, tel que le diméthylformamide et l'on traite le sel obtenu par une base forte : hydroxyde alcalin en milieu aqueux ou alcoolate en milieu alcoolique, pour isoler l'ylide de formule I.

L'ylide (I) est ensuite mis à réagir dans les conditions classiques de la réaction de Wittig avec le propionaldéhyde. La condensation est effectuée sans solvant ou dans un éther, par exemple, dans le dioxane ou le tétrahydrofuranne, de préférence à une température supérieure à la température ambiante et même proche de la température de reflux du solvant, éventuellement sous une légère pression, inférieure à $5 \times 10^5$ Pa. La purification de l'ester est effectuée par distillation sous pression réduite, dans un ap pareil classique; de préférence, la pression est comprise entre 1000 Pa et 5000 Pa.

Pour obtenir l'acide, l'hydrolyse de la fonction ester est réalisée en milieu acide ou basique hydroalcoolique, de préférence en milieu basique. L'acide brut est cristallisé dans l'éther de pétrole refroidi à une température inférieure à - 10 °C.

La stéréochimie de l'acide obtenu a été étudiée en résonance magnétique nucléaire avec effet Overhauser Nucléaire en présence de terre rare.

On a ainsi déterminé que le produit était en configuration E selon les règles de Ingold et pouvait donc être représenté par la formule développée :

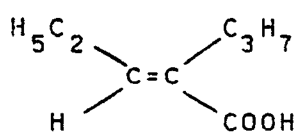

Dans ce qui suit, on décrit des exemples de réalisation de l'invention.

## EXEMPLE 1

a) bromure d'(éthoxycarbonyl-1) butyl-1 triphényl phosphonium (formule IV, $R_1 = C_2H_5$, $R_2 = C_6H_5$)

On introduit 3650g de bromo-2 valérate d'éthyle de 4671g de triphénylphosphine dans 101 de diméthylformamide et maintient le mélange à 80 °C pendant 24 heures; après élimination des produits volatils par distillation sous pression réduite, on fait cristalliser le résidu dans l'éther diisopropylique pour isoler 6912g de sel de phosphonium.

Rendement : 84%.

b) (éthoxycarbonyl-1) butène-1 triphényl phosphorane (formule I, $R_1 = C_2H_5$, $R_2 = C_6H_5$)

On introduit dans un mélange agité de 21l d'eau et 21l de dichlorométhane, 6907g du sel précédemment obtenu, puis peu à peu 1030ml de solution aqueuse de NaOH (33% p/v). Après 30 minutes d'agitation, on sépare la phase organique et évapore le solvant sous pression réduite après son lavage à l'eau et sa déshydratation par $Na_2SO_4$. On verse 11l d'hexane sur le résidu et isole le précipité; après séchage, il pèse 5283g.

Rendement : 92,3%.

c) (propyl-2) pentène-2 oate d'éthyle (formule A, $R_1 = C_2H_5$)

On dissout 5220g de l'ylide obtenu selon b) dans 8,5l de dioxanne puis 2,9l de propionaldéhyde. Après 24 heures à la température de reflux, l'aldéhyde en excès et le solvant sont distillés sous pression réduite et on verse 10l d'éther éthylique sur le résidu. Le précipité formé, d'oxyde de triphénylphosphine, est séparé et le solvant est éliminé sous pression réduite; l'ester est ensuite distillé à 90-92 °C sous 2700 Pa. On obtient 1492g d'ester en configuration E en mélange avec 4% d'ester, en configuration Z.

Rendement par rapport à l'ester alpha bromé : 51%.

Spectre RMN : (250MHz-étalon TMDS; solvant $CDCl_3$) 6,7 ppm (triplet pour 1H); 4,2 ppm (quadruplet pour 2H); 2,4 ppm (quadruplet pour 4H); 1 ppm (multiplet pour 11H).

EXEMPLE 2

(propyl-2) pentène-2 oate d'éthyle

On maintient à 80 °C pendant 24 heures, sous une pression de $3 \times 10^5$ Pa, un mélange de 728g d'ylide obtenu en appliquant le procédé décrit à l'exemple 1b et 830 ml de propionaldéhyde. L'excès d'aldéhyde est ensuite éliminé par distillation sous pression ré duite; le précipité formé après addition de 6l d'éther diisopropylique sur le résidu est éliminé, et le solvant puis l'ester cherché sont distillés sous pression réduite. On isole ainsi 196g d'ester.

Rendement : 48% par rapport à l'ester alpha bromé.

EXEMPLE 3

Acide (propyl-2)pentène-2 oïque (isomère E)

On introduit 1466g d'ester préparé selon les exemples 1 ou 2 dans une solution de 4620g de KOH dans 10,6l d'éthanol aqueux (50/50-v/v).

Après 75 minutes à la température de reflux, on laisse le milieu revenir à température ambiante. Il est ensuite versé lentement sous agitation sur un mélange de 20 kg de glace pilée et 9l de solution aqueuse d'acide chlorhydrique 10N. Lorsque l'ensemble est revenu à température ambiante, on extrait deux fois l'acide de la phase aqueuse dans 9l d'hexane; les phases organiques sont réunies, lavées, séchées et le solvant est distillé sous pression réduite.

On verse sur le résidu 1l d'éther de pétrole et isole le précipité apparu après quelques heures à -20 °C. Il est constitué de 1024g de l'acide cherché en configuration E qui fond à 33 °C.

Rendement : 83,6%.

Spectre RMN : (250MHz-solvant : $CDCl_3$ étalon interne TMDS) 12,3 ppm (singulet pour 1H); 6,8 à 7,1 ppm (triplet pour 1H); 2 à 2,5 ppm (multiplet pour 4H); 1,2 à 1,7 ppm (multiplet pour 2H); 0,7 à 1,2 ppm (multiplet pour 6H).

On a préparé divers sels de l'acide E (propyl-2)pentène-2 oïque.

. Sel de potassium :

20g d'acide propyl-2 pentène-2 oïque sont dissous dans 200 ml d'éthanol; on ajoute une solution éthanolique de 8,0g de potasse en pastilles dans 88 ml d'éthanol.

Après 1 heure d'agitation à température ambiante, on élimine le solvant le résidu est repris à l'acétone et on évapore à sec. Le sel de potassium de l'acide propyl-2 pentène-2 oïque, isomère E, concrétise dans l'hexane : on obtient 9,44g de produit.

. Sel de magnésium :

On dissout à 50° C, 7,1g d'acide propyl-2 pentène-2 oïque, isomère E, et 1,456g d'hydroxyde de magnésium, dans 250ml d'eau : on lyophilise la solution obtenue et le résidu cristallise dans l'acétone. On obtient 4,5g de sel de magnésium de l'acide propyl--2 pentène-2 oïque, isomère E.

. Sel de calcium :

On mélange 5g de propyl-2 pentène-2 oate de sodium, isomère E, dans 20 ml d'eau bouillie, on additionne une solution de 2,29g de chlorure de calcium, dihydrate dans 10 ml d'eau bouillie, en présence d'un antioxydant (hydroquinone). Le précipité obtenu est filtré et lavé à l'eau et à l'acétone. On obtient 2,85g de propyl-2 pentène-2 oate de calcium, isomère E.

**Revendications**

1. Procédé de préparation des isomères E des composés de formule A

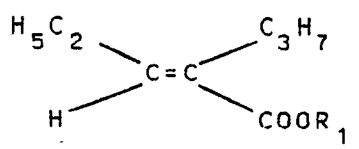

A

dans laquelle $R_1$ représente H ou un groupe alkyle en $C_1$ à $C_4$, caractérisé en ce qu'on fait réagir le propionaldéhyde sur un dérivé phosphoré de formule

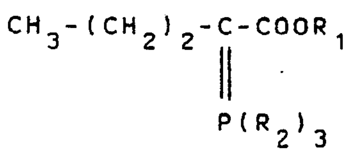

I

dans laquelle $R_1$ désigne un groupe alkyle en $C_1$ à $C_4$ et $R_2$ désigne un groupe alkyle en $C_1$ à $C_4$ ou un groupe hényle, et le cas échéant on hydrolyse le composé de formule A dans laquelle $R_1$ représente un groupe alkyle pour obtenir le composé de formule A dans laquelle $R_1$ représente H.

2. Procédé selon la revendication 1, caractérisé en ce que la réaction est effectuée dans un solvant aprotique polaire à une température comprise entre 20° C et 100° C.

3. Procédé selon la revendication 2, caractérisé en ce que la réaction est effectuée dans le dioxanne au reflux.

4. Procédé selon l'une des revendications 1 à 3, caractérisé en ce que l'hydrolyse de l'ester de formule A est effectuée en milieu hydroalcoolique basique.

5. Procédé selon l'une des revendications précédentes, caractérisé en ce que l'ester est distillé sous pression réduite avant l'hydrolyse et que l'acide est cristallisé dans l'éther de pétrole à une température inférieure à -10° C.

Office européen des brevets

# RAPPORT DE RECHERCHE EUROPEENNE

Numero de la demande

EP 89 40 3346

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | THE JOURNAL OF ORGANIC CHEMISTRY vol. 51, no. 9, 2 mai 1986, pages 1735-1741, American Chemical Society; J.A. MARSHALL et al.: "Condensation of Long-Chain alpha-Phosphono Carboxylates with Aldehydes" * page 1736, tableau 1; page 1739, tableau 3; résumé * | 1,2 | C 07 C 51/347<br>C 07 C 57/03<br>C 07 C 67/347<br>C 07 C 69/533 |
| A,D | CHEMISCHE BERICHTE vol. 99, no. 4, 1966, pages 1198-1207, Verlag Chemie GMBH; H.-J. BESTMANN et al.: "Eine neue Synthese von Allen-carbonsäureestern" * page 1203 * | 1,2 | |
| A,D | CHEMICAL REVIEWS vol. 74, no. 1, 1974, pages 87-99; J. BOUTAGY et al.: "Olefin Synthesis with Organic Phosphonate Carbanions" * pages 87-92 * | 1,2 | |
| A | BULLETIN OF THE CHEMICAL SOCIETY OF JAPAN vol. 40, no. 12, décembre 1967, pages 2968-1970; K. SASAKI et al.: "Synthesis of Ethyl 5,9-Dimethyl-2,4,8-decatrienoates and the Homologs" | 1,2 | **DOMAINES TECHNIQUES RECHERCHES (Int. Cl.5)**<br>C 07 C 51/00<br>C 07 C 57/00<br>C 07 C 67/00<br>C 07 C 69/00 |
| A | BEILSTEINS HANDBUCH DER ORGANISCHEN CHEMIE 4ième édition, vol. 2, 1920, Julius Springer Verlag, Berlin, RFA * page 452 * | 1 | |
| E | EP-A-0 293 753 (DESITIN ARNEIMITTEL GMBH) * revendication 1; example 2 * | 1,4,5 | |

Le présent rapport a été établi pour toutes les revendications

| Lieu de la recherche | Date d'achèvement de la recherche | Examinateur |
|---|---|---|
| BERLIN | 06-02-1990 | RUFET J.M.A. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)